# EUROPEAN PATENT SPECIFICATION

(11) **EP 3 189 139 B1**
(45) Date of publication and mention of the grant of the patent: **14.08.2019**
(21) Application number: 15759414.4
(22) Date of filing: 18.08.2015
(51) Int. Cl.: C12N 15/10

(54) **A PROCESS FOR SEPARATION OF PROKARYOTIC DNA FROM EUKARYOTIC DNA IN A WHOLE BLOOD SAMPLE**
VERFAHREN ZUR TRENNUNG VON PROKARYOTISCHEM DNA VON EUKARYOTISCHEM DNA IN EINER VOLLBLUTPROBE
PROCÉDÉ POUR LA SÉPARATION D'ADN PROCARYOTE D'ADN EUCARYOTE DANS UN ÉCHANTILLON DE SANG TOTAL

(30) Priority: 03.09.2014 IN 909KO2014
(43) Date of publication of application: 12.07.2017
(73) Proprietor: Siemens Healthcare Diagnostics Inc., Tarrytown, NY 10591 (US)
(72) Inventor: RUKMINI JAYARAMAN, Subhadra, Bangalore, Karnataka 560095 (IN); VUTUKURU, Ramya, Bangalore, Karnataka 560019 (IN)
(74) Representative: Patentanwälte Bals & Vogel
(86) International application number: PCT/EP2015/068936
(87) International publication number: WO 2016/034405

(56) References cited:
- US-A1- 2013 017 552
- US-A1- 2014 127 687
- US-B2- 8 481 262
- US-B2- 8 481 262

## Description

The disclosure relates to a process for separation of prokaryotic DNA from eukaryotic DNA in a given whole blood sample infected by pathogen, said process comprising of the steps involving extracting prokaryotic DNA and eukaryotic DNA from said whole blood sample, contacting said prokaryotic DNA in said whole blood sample with a motif that specifically binds to said prokaryotic DNA and generating a bound pair, and separating said bound pair.

Such a process is already disclosed in US 8,481,262. The process disclosed therein involves contacting at least one prokaryotic DNA with a protein that is specific to said prokaryotic DNA and thus forming a protein-DNA complex. Said protein is 25% to 35% homologous to wild type CGPB protein. Such protein disclosed therein binds to non-methylated CpG motifs in the prokaryotic DNA. Such protein-DNA complex disclosed therein is separated and said prokaryotic DNA is further separated from said protein-DNA complex. The process disclosed therein is based on the principle that prokaryotic DNA is non-methylated and thus, a protein specific to non-methylated CpG motifs in the prokaryotic DNA is used to separate said prokaryotic DNA. The process disclosed therein may not be specific to prokaryotic DNA as eukaryotic DNA may be non-methylated under conditions like cancer and old age. Under such conditions, certain motifs of the eukaryotic DNA may be non-methylated and may get separated along with said prokaryotic DNA in the given sample.

The object of the disclosure is therefore to provide a process of a kind mentioned in the introduction which is more efficient and specific in separation of prokaryotic DNA from eukaryotic DNA and to bring forth higher recovery of the required prokaryotic DNA during the separation process.

The invention achieves this object in that the whole blood sample is transfected with bacteriophages genetically modified to contain a defined DNA sequence having a selectable marker DNA, wherein said DNA sequence integrates into said prokaryotic DNA, and said motif is an oligonucleotide complementary to said defined DNA sequence in said engineered bacteriophages.

The present disclosure provides for a process that is more efficient and specific that which provides higher recovery of prokaryotic DNA as compared to the method disclosed in US 8,481,262. The object of the present invention is achieved by a process for separation of prokaryotic DNA from eukaryotic DNA in a whole blood sample. According to the process, whole blood sample is collected from a patient to be tested using a needle and the DNA from said whole blood sample infected by pathogen is extracted such that said prokaryotic DNA is also extracted along with the eukaryotic DNA. Whole blood sample refers to blood drawn from human body from which no constituents such as platelets or plasma have been removed. A prokaryotic DNA refers to the DNA of a single cellular organism which does not have a membrane bound nucleus. A eukaryotic DNA refers to the DNA of an organism whose cells have a nucleus and other organelles bound within membranes. The extraction of DNA ensures easier detection and separation of pathogenic prokaryotic DNA from the given whole blood sample.

According to the invention, the extracted prokaryotic DNA is brought in contact with a motif that specifically binds to said prokaryotic DNA. A motif, according to this invention, refers to a sequence pattern of nucleotides in a DNA sequence. Once said prokaryotic DNA binds to said motif a bound pair is generated. Such bound pair is separated for further analysis.

According to the invention, said whole blood sample is transfected with bacteriophages that have been genetically modified to contain a defined DNA sequence containing a selectable marker DNA, wherein such a DNA sequence integrates into said prokaryotic DNA. Transfection is a process by which a virus or a bacteriophage introduces a DNA or RNA molecule into bacterial cells resulting in infection. A selectable marker DNA is a DNA sequence used in molecular biology to ensure that a particular DNA sequence has been inserted into an organism's DNA.

According to the invention, said motif used for separation of said prokaryotic DNA is an oligonucleotide that is specific to said defined DNA sequence in said engineered bacteriophages. Hence, the complex formed between said oligonucleotide and said defined DNA sequence helps in isolating said prokaryotic DNA in which said defined DNA sequence has been integrated from eukaryotic DNA.

According to an embodiment of the invention, said whole blood sample is transfected with said engineered bacteriophages before genomic DNA is extracted from said whole blood sample. Thus, when said eukaryotic DNA along with said prokaryotic DNA is extracted, the defined DNA sequence has already been integrated into said prokaryotic DNA. This step allows detection and separation of said prokaryotic DNA to be easier and more efficient.

According to the preferred embodiment of the invention, said pathogen to be detected in said whole blood sample is a bacterium as bacteriophages transfect bacteria.

According to another preferred embodiment of the invention, said prokaryotic DNA is bacterial DNA.

According to another preferred embodiment of the invention, said bacteriophage engineered and used for transfection of said pathogenic bacteria is a lysogenic bacteriophage. A bacteriophage undergoing lytic cycle of reproduction on transfection would degrade the bacterial DNA. In order to make certain that the bacterial DNA is intact and the phage genome integrates into such bacterial DNA, bacteriophage employing lysogenic cycle of reproduction is chosen.

According to another preferred embodiment of the invention, said oligonucleotide is bound to a carrier. The binding of the oligonucleotide to a carrier facilitates its immobilization. Said carrier is an adsorbent micro particle, membrane or a matrix and allows the oligonucleotide to bind to its surface.

According to another embodiment of the invention, said prokaryotic DNA is separated from the said oligonucleotide of said bound pair after separation of said bound pair. This ensures that said prokaryotic DNA is made available in its pure form for further experiments. According to a preferred embodiment of the invention, such separation of said prokaryotic DNA from oligonucleotide of said bound pair can be achieved by means of an elution buffer. Elution buffer is a solvent that at higher concentrations releases the desired motif from the ligand.

According to another embodiment of the invention, said eukaryotic DNA is human genomic DNA.

The present invention is further described hereinafter with reference to illustrated embodiments shown in the accompanying drawings, in which:
- FIG 1: illustrates a schematic diagram of a genome of an engineered bacteriophage containing defined DNA sequence along with a selectable marker DNA.
- FIG 2: illustrates a schematic diagram of the process for separation of prokaryotic DNA from eukaryotic DNA from a whole blood sample infected by pathogen.
- FIG 3: illustrates a flowchart of an embodiment the method according to the invention.

Hereinafter, embodiments for carrying out the present invention are described in detail. The various embodiments are described with reference to the drawings, wherein like reference numerals are used to refer to like elements throughout. In the following description, for purpose of explanation, numerous specific details are set forth in order to provide a thorough understanding of one or more embodiments. It may be evident that such embodiments may be practiced without these specific details.

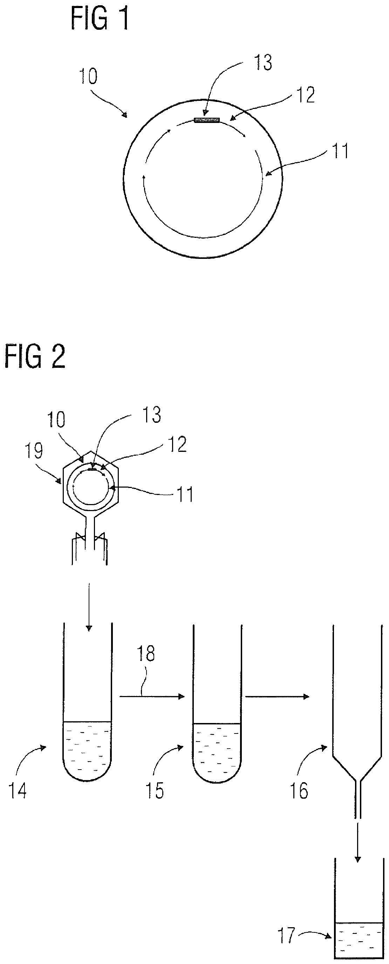

FIG 1 illustrates the genomic DNA of an engineered bacteriophage 10. In an embodiment of the invention as shown in FIG 1, the bacteriophage 10 that employs lysogenic cycle of reproduction is chosen and is genetically engineered. Lysogenic cycle of reproduction is one of the ways of reproduction wherein the nucleic acid of the bacteriophage 10 is integrated into the genome of the bacterium it infects. The viral genome thus propagates along with the bacterial DNA when the bacterium undergoes genetic division. In the lysogenic phase, the bacterial cells are not lysed contrarily to lytic cycle of viral reproduction. Said lysogenic bacteriophage may be chosen such that it is capable of transfecting different types of pathogenic bacteria infecting said whole blood sample, for example, M13 phage delivery system. However, a concoction of multiple bacteriophages for transfecting the possible pathogens present in the given whole blood sample may be used. In the embodiment of the invention, genomic DNA of bacteriophage 10 may be modified to include a defined DNA sequence 12 integrated within bacteriophage genomic DNA 11. Said defined DNA sequence 12 to be introduced into the bacteriophage 19 comprises of balanced nucleobase content along with a selectable marker DNA 13. In an embodiment of the invention, said selectable marker DNA 13 may be an antibiotic resistance gene. A selectable marker DNA is a nucleotide sequence that helps in ensuring success of introduction of a foreign DNA into a cell. Hence, said antibiotic resistance gene helps in choosing bacterial cells that have said defined DNA sequence in their genomic DNA.

FIG 2 illustrates the process for separation of prokaryotic DNA from eukaryotic DNA in a whole blood sample infected by pathogen. In an embodiment of the invention as shown in FIG 2, said genetically modified lysogenic bacteriophage 19 is added to the whole blood sample 14 collected from a patient. Whole blood refers to blood drawn from human body from which no constituents such as platelets or plasma have been removed. The engineered bacteriophages 19, in the range of 10⁶ and 10⁹ PFU in approximately 200uL saline, are inoculated in said whole blood sample 14 by methods known to persons skilled in the art. Said engineered bacteriophages 19 transfect pathogenic bacteria present in said whole blood sample 14 and start the lysogenic cycle. Thus said engineered genome 10 integrates into the bacterial genome. Said bacterial genome is the prokaryotic DNA of interest which is separated. Transfection is a process in which a prokaryotic virus or a bacteriophage infects a bacterium and transfers the viral DNA into the bacterium.

In the next step 18 of the invention, genomic DNA is extracted from said whole blood sample 14 such that the prokaryotic DNA from the pathogenic bacteria is also released. The extraction methods used are well known to persons skilled in the art. A lysing mixture added to said whole blood sample 14, for the process of extraction of genomic DNA, releases the internal cellular components of said pathogenic bacteria. Such lysing mixture also breaks down the blood cells in the sample and releases a substantial amount of eukaryotic DNA.

In the next step of the invention, said prokaryotic DNA is separated from said eukaryotic DNA released in the extraction process. In an embodiment of the invention, an oligonucleotide, specific to said defined DNA sequence integrated within said prokaryotic DNA, bound to a carrier molecule is used for separation of said prokaryotic DNA from said whole blood sample 14. Said carrier molecule is an adsorbent and may be a micro-particle, membrane or matrix so as to facilitate binding of said oligonucleotide on its surface. The oligonucleotide binds to said prokaryotic DNA and forms a bound pair complex. Such bound pair complex helps in separation 16 of said prokaryotic DNA from eukaryotic DNA and other cellular components released in said blood sample. In the next step of the invention, said prokaryotic DNA is separated 17 from said oligonucleotide of said bound pair complex.

Various methods may be employed to separate 16 said bound pair complex of said prokaryotic DNA and said oligonucleotide. Such methods are explained in detail as examples, but not limiting the invention thereto.

### Example 1: Separation by Affinity Chromatography

In an embodiment of the invention, affinity chromatography method may be used for such separation of said prokaryotic DNA. In such embodiment, carrier molecules bound to said oligonucleotides having affinity towards said prokaryotic DNA is introduced in a column 16. The lysed mixture 15 containing extracted genomic DNA and prokaryotic DNA is passed through such column and the flow through obtained is collected. Said collected flow through is passed through second identical column containing carrier molecules bound to said oligonucleotides. Said prokaryotic DNA of interest in said whole blood sample binds to said oligonucleotides in the columns, allowing eukaryotic DNA and other cellular components to flow through said columns. Said columns are washed with a wash buffer to remove any unspecific DNA or debris bound to the columns. An elution buffer may be used to release 17 said bound prokaryotic DNA from said oligonucleotide. The buffer releases said prokaryotic DNA from the carrier by binding to said carrier in place of said prokaryotic DNA.

### Example 2: Separation by Lateral Flow Assay

Lateral flow assay is a method which helps in detection and separation of an analyte of interest from given sample. In an embodiment of the invention, said lysed whole blood sample 15 containing eukaryotic DNA and prokaryotic DNA is added on a lateral flow strip 16 consisting of a conjugate pad, a nitrocellulose membrane and an absorbent pad. Said conjugate pad contains gold nano-particle probes having complementary sequences to said defined DNA sequence 12 integrated into said prokaryotic DNA. Said nitrocellulose membrane contains two zones, detection zone and control zone. The detection zone contains said oligonucleotides complementary to said prokaryotic DNA, whereas control zone contains an oligonucleotide sequence specific to gold nano-particle probe. The absorbent pad absorbs the excess of the sample.

When said lysed blood sample 15 is added on the lateral flow strip, said prokaryotic DNA binds with the gold nano-particle probe and flows down the strip. Such complex formed between said prokaryotic DNA and gold nano-particle probe binds to said oligonucleotide present in said detection zone of said nitrocellulose membrane. The unbound gold nano-particles flow down and bind to said control zone of said nitrocellulose membrane. Said prokaryotic DNA bound to gold nano-particle probe and oligonucleotide in said detection zone can be eluted 17 using an elution buffer.

### Example 3: Biotin based magnetic separation

Biotin is an affinity molecule that is widely used for detection and separation of biological molecules. It has a very strong affinity for streptavidin protein and thus this pair can be used for separation of analytes of interest. In an embodiment of the invention, said oligonucleotide sequence complementary to said prokaryotic DNA to be separated is labeled with biotin. Once said lysed whole blood sample 15 is treated with said biotinylated oligonucleotide said prokaryotic DNA in said whole blood sample 14 binds to such oligonucleotide and forms a complex. In the embodiment of the invention, said whole blood sample with said complex is exposed to streptavidin molecules bound to magnetic beads. In the presence of streptavidin molecule, biotinylated oligonucleotide along with said prokaryotic DNA binds to it. Such a complex formed can be separated by application of a magnetic field. Thus, said prokaryotic DNA can be separated from said eukaryotic DNA and other cellular components.

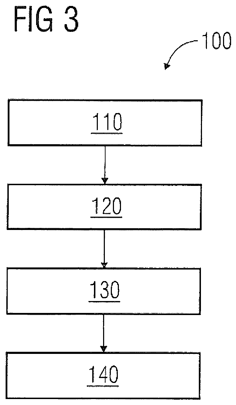

FIG 3 is a process flowchart illustrating an embodiment of the process for separation of prokaryotic DNA from eukaryotic DNA from a whole blood sample infected by pathogen. The process 100 consists of the following steps. At step 110, genetically engineered bacteriophages 19 are added to said whole blood sample 14 so as to allow said engineered bacteriophages 19 to infect the bacteria in said whole blood sample 14. At step 120, eukaryotic and prokaryotic DNA from said whole blood sample 14 is extracted. At step 130, said prokaryotic DNA in said lysed whole blood sample 15 is separated 16 from said eukaryotic DNA by adding said lysed whole blood sample 15 to carrier molecules bound to oligonucleotides specific to said defined DNA sequence 12 integrated into said prokaryotic DNA. Said defined DNA sequence 12 binds to said oligonucleotides on said carrier molecules and form a bound pair complex. At step 140, said bound pair complex is eluted out 17 using an elution buffer.

## Claims

1. A process (100) for separation of prokaryotic DNA from eukaryotic DNA in a given whole blood sample (14) infected by a bacterium, said process comprising of the following steps:
(a) extracting prokaryotic and eukaryotic DNA from said whole blood sample (14);
(b) contacting lysed whole blood sample (15) comprising said extracted prokaryotic and eukaryotic DNA with a motif that specifically binds to said prokaryotic DNA and generating a bound pair;
(c) separating said bound pair;
**characterized in that**:
said whole blood sample (14) is transfected with bacteriophages (19) genetically modified to contain a defined DNA sequence (12) having a selectable marker DNA (13), wherein said DNA sequence (12) integrates into said prokaryotic DNA; wherein said whole blood sample (14) is transfected with said bacteriophages (19) before genomic DNA is extracted from the whole blood sample (14);
said motif is an oligonucleotide complementary to said defined DNA sequence (12) in said engineered bacteriophages (19).

2. The process (100) of Claim 1 wherein: said bacteriophage (19) is a lysogenic bacteriophage.

3. The process (100) of Claim 1 wherein: said oligonucleotide is bound to a carrier.

4. The process (100) of Claim 3 wherein: said carrier is an adsorbent micro-particle, a membrane or a matrix.

5. The process (100) of Claim 1 wherein: separating said bound pair is followed by separating said prokaryotic DNA from said oligonucleotide of said bound pair.

6. The process (100) of Claim 5 wherein: said separation of prokaryotic DNA from said oligonucleotide is achieved by means of an elution buffer.

7. The process (100) of Claim 1 wherein: said eukaryotic DNA is human genomic DNA.

## Patentansprüche

1. Verfahren (100) zur Trennung von prokaryotischer DNA von eukaryotischer DNA in einer gegebenen Vollblutprobe (14), die von einem Bakterium infiziert ist, wobei das Verfahren die folgenden Schritte umfasst:
(a) Extrahieren prokaryotischer und eukaryotischer DNA aus der Vollblutprobe (14);
(b) Kontaktieren einer lysierten Vollblutprobe (15), umfassend die extrahierte prokaryotische und eukaryotische DNA mit einem Motiv, das spezifisch an die prokaryotische DNA bindet und ein gebundenes Paar erzeugt;
(c) Trennen des gebundenen Paares;
**dadurch gekennzeichnet, dass**:
die Vollblutprobe (14) mit Bakteriophagen (19) transfiziert wird, die genetisch modifiziert sind, um eine definierte DNA-Sequenz (12) mit einer wählbaren Marker-DNA (13) zu enthalten, worin die DNA-Sequenz (12) in die prokaryontische DNA integriert ist; wobei die Vollblutprobe (14) mit den Bakteriophagen (19) transfiziert wird, bevor genomische DNA aus der Vollblutprobe (14) extrahiert wird;
das Motiv ein Oligonukleotid ist, das komplementär zu der definierten DNA-Sequenz (12) in den konstruierten Bakteriophagen (19) ist.

2. Verfahren (100) nach Anspruch 1, wobei:
der Bakteriophage (19) ein lysogener Bakteriophage ist.

3. Verfahren (100) nach Anspruch 1, wobei:
das Oligonukleotid an einen Träger gebunden ist.

4. Verfahren (100) nach Anspruch 3, wobei:
der Träger ein adsorbierendes Mikroteilchen, eine Membran oder eine Matrix ist.

5. Verfahren (100) nach Anspruch 1, wobei:
das Trennen des gebundenen Paares gefolgt ist von dem Trennen der prokaryotischen DNA von dem Oligonukleotid des gebundenen Paares.

6. Verfahren (100) nach Anspruch 5, wobei:
das Trennen der prokaryotischen DNA vom Oligonukleotid mittels eines Elutionspuffers erreicht wird.

7. Verfahren (100) nach Anspruch 1, wobei:
die eukaryotische DNA eine genomische DNA des Menschen ist.

## Revendications

1. Procédé (100) de séparation d'ADN procaryote de l'ADN eucaryote dans un échantillon de sang total donné (14) infecté par une bactérie, ledit procédé comprenant les étapes consistant à :
(a) extraire l'ADN procaryote et eucaryote dudit échantillon de sang total (14) ;
(b) mettre en contact un échantillon de sang total lysé (15), comprenant ledit ADN procaryote et eucaryote extrait avec un motif qui se lie spécifiquement audit ADN procaryote, et produire une paire liée ;
(c) séparer ladite paire liée ;
**caractérisé en ce que** :
ledit échantillon de sang total (14) est transfecté avec des bactériophages (19) génétiquement modifiés pour contenir une séquence d'ADN définie (12) ayant un ADN marqueur sélectionnable (13), ladite séquence d'ADN (12) s'intégrant dans ledit ADN procaryote ; ledit échantillon de sang total (14) étant transfecté avec lesdits bactériophages (19) avant que l'ADN génomique ne soit extrait de l'échantillon de sang total (14) ;
ledit motif est un oligonucléotide complémentaire de ladite séquence d'ADN définie (12) dans lesdits bactériophages modifiés (19).

2. Procédé (100) selon la revendication 1, dans lequel :
ledit bactériophage (19) est un bactériophage lysogène.

3. Procédé (100) selon la revendication 1, dans lequel :
ledit oligonucléotide est lié à un support.

4. Procédé (100) selon la revendication 3, dans lequel :
ledit support est une microparticule adsorbante, une membrane ou une matrice.

5. Procédé (100) selon la revendication 1, dans lequel :
la séparation de ladite paire liée est suivie de la séparation entre ledit ADN procaryote et ledit oligonucléotide de ladite paire liée.

6. Procédé (100) selon la revendication 5, dans lequel :
ladite séparation de l'ADN procaryote dudit oligonucléotide est réalisée au moyen d'un tampon d'élution.

7. Procédé (100) selon la revendication 1, dans lequel :
ledit ADN eucaryote est un ADN génomique humain.
